# EUROPÄISCHE PATENTSCHRIFT

(11) **EP 0 732 964 B1**
(45) Veröffentlichungstag und Bekanntmachung des Hinweises auf die Patenterteilung: **13.08.1997**
(21) Anmeldenummer: 95903750.8
(22) Anmeldetag: 05.12.1994
(51) Int. Cl.: A62D 3/00, C12N 1/00, B09C 1/10, C02F 3/34

(54) **VERFAHREN UND MITTEL ZUR DEKONTAMINIERUNG MITTELS MIKROORGANISMEN**
PROCESS AND AGENT FOR THE DECONTAMINATION BY MEANS OF MICRO-ORGANISMS
PROCEDE ET AGENT PERMETTANT LA DECONTAMINATION AU MOYEN DE MICRO-ORGANISMES

(30) Priorität: 06.12.1993 DE 4341339
(43) Veröffentlichungstag der Anmeldung: 25.09.1996
(73) Patentinhaber: EISU INNOVATIVE GESELLSCHAFT FÜR TECHNIK UND UMWELTSCHUTZ MBH, 67759 Wolfen (DE); TDA Technische Dienste und Anlagenbau GmbH, 06803 Greppin (DE); Kludas, Uwe, 06849 Dessau (DE)
(72) Erfinder: KLUDAS, Uwe, D-06849 Dessau (DE); KLUDAS, Karl-Heinz, D-06846 Dessau (DE); MÖBIUS, Günther, D-06786 Vockerode (DE); HARMS, Uwe, D-06846 Dessau (DE); LANGE, Eckehard, D-06846 Dessau (DE)
(74) Vertreter: Tragsdorf, Bodo, Dipl.-Ing.
(86) Internationale Anmeldenummer: DE9401441
(87) Internationale Veröffentlichungsnummer: WO9515788

(56) Entgegenhaltungen:
- EP-A- 0 493 688
- US-A- 3 262 861
- US-A- 4 853 334
- US-A- 5 209 851

## Beschreibung

Die Erfindung betrifft ein Verfahren zur Dekontaminierung von in Feststoffen oder Flüssigkeiten enthaltenen organischen Schadstoffen, insbesondere solchen, die nur schwer abbaubar sind, mittels Mikroorganismen, insbesondere solchen, die am Standort bereits vorhanden sind. Die Erfindung betrifft ferner ein geeignetes Mittel zur Durchführung des Verfahrens.

Es ist bekannt, zur Sanierung von Böden, Bodenkontaminationen und Abwässer unterschiedliche Verfahren, wie z.B. thermische Verfahren, Waschverfahren und mikrobiologische Verfahren, anzuwenden. Thermische Behandlungsverfahren und Wäschen erfordern einen aufwendigen Bodenaushub, sind energieaufwendig und teuer.
Kostengünstigere Sanierungen werden mit biologischen Verfahren erreicht. Das Prinzip der mikrobiologischen Bodenbehandlung beruht bekanntlich auf einer kontrollierten Nutzung des natürlichen Potentials von Mikroorganismen zur Umsetzung von vielfältigen organischen Verbindungen. Die Verfahrenspraxis mikrobiologischer Sanierungen besteht in einer gezielten Förderung der natürlichen Abbauleistungen der Mikroorganismen durch Einstellung günstiger Umweltbedingungen.
Es ist bekannt, daß dazu eine optimale Versorgung mit Nährstoffen, Wasser, Elektronenakzeptoren bzw. ausreichende Belüftung sowie die Einstellung geeigneter Temperaturen und pH-Werte gehören. Normalerweise sind die Standortfaktoren bei sanierungsbedürftigen Bodenkontaminationen nicht günstig, so daß der Schadstoffabbau mit gezielten Maßnahmen unterstützt werden muß.
Über die mikrobiologische Abbaubarkeit verschiedener Schadstoffe liegt eine große Anzahl von Untersuchungen vor. So ist bekannt, Bodenkontaminationen durch kurzkettige n-Alkane, einfache Cycloalkane, Phenole, Aromaten, wie z.B. Benzol, Toluol und Xylol, sowie einige einfache chlorierte Kohlenwasserstoffe, mikrobiologisch zu sanieren.

Im Gegensatz dazu gelten polycylische aromatische Kohlenwasserstoffe (PAK), Nitrophenole, Chlorphenole und im besonderen die höher substituierten chlorierten Aromaten, wie z.B. polychlorierte Biphenyle, polychlorierte Dibenzodioxine und -furane, als schwer abbaubar. Für einen großen Teil dieser umweltrelevanten organischen Verbindungen sind die mikrobiellen Abbaumechanismen bekannt, jedoch gibt es für diese Verbindungen gegenwärtig noch keine kostengünstigen, wirtschaftlichen Sanierungsverfahren im großtechnischen Maßstab, da ihr Abbau Zeiträume von mehreren Jahren erfordert.
Aus der DE 31 39 756 C2 ist ein Verfahren bekannt, wonach Erdölschlamm mit Holzabfällen kompostiert wird. Die im Erdölschlamm enthaltenen Bakterien zersetzen jedoch das Erdöl mit Holzabfällen als alleiniger Nährstoffquelle nur sehr langsam.

Der Erfindung lag die Aufgabe zugrunde, ein Verfahren zur Dekontaminierung von in Feststoffen oder Flüssigkeiten enthaltenen organischen Schadstoffen mittels Mikroorganismen zu schaffen, das den Abbau, insbesondere als persistent geltender Schadstoffe, wie z.B. PAK, Nitrophenole, Chlorphenole und im besonderen höher substituierter chlorierter Aromaten, in wesentlich kürzerer Zeit als die bisher bekannten Verfahren ermöglicht, universell einsetzbar ist und sich durch eine hohe Wirtschaftlichkeit auszeichnet.
Ferner war es Aufgabe der Erfindung, ein geeignetes Mittel zur Durchführung des Verfahrens bereitzustellen.

Erfindungsgemäß wird die Aufgabe durch die in den Verfahrensansprüche 1 bis 12 genannten Merkmale gelöst. Ein geeignetes Mittel zur Durchführung des Verfahrens ist in den Ansprüchen 13 bis 16 angegeben und im Anspruch 17 eine Verwendung eines Mittels gemäß Anspruch 13.
Das vorgeschlagene Mittel stellt einen Biokomplex dar und dient den schadstoffabbauenden Mikroorganismen in Verbindung mit den organischen Schadstoffen als optimale Nährstoffquelle. Es dient als Energie-, Phosphor- und Stickstoffquelle und liefert Vitamine und Spurenelemente zum optimalen und aktuellen biologischen Bedarf. Durch gezielte Minimierung des Kohlenstoffanteils im Mittel wird die Voraussetzung zum Schadstoffabbau durch Bakterien und Pilze, denen die organischen Schadstoffe als Kohlenstoff-Quelle dienen sollen, geschaffen.
Der Abbau organischer Schadstoffe erfolgt durch Bakterien-/Pilzgemische mit heterotropher Assimilation unter aeroben oder anaeroben Bedingungen. Zu dieser Ernährungsform werden organische Stoffe zumindest als Energie- aber auch als Stickstoff-quelle benötigt. Die proteinogene Stickstoffquelle garantiert u.a. eine hohe Dichte an Mikroorganismen. Sie ist die Voraussetzung für einen schnellen und umfassenden Abbau organischer Schadstoffe. Die in dem Mittel enthaltenen Bestandteile garantieren sowohl die Dekontaminierung leicht als auch schwer abbaubarer Schadstoffe, wie z.B. PCB, PAK und Chlorphenole.
Die Bestandteile des erfindungsgemäßen Mittels basieren zu 100 % auf natürlichen Substanzen. Es erfolgt deshalb keine ökotoxikologische Beeinträchtigung der Bodenqualität durch ungenutzte Nähr- oder Stoffwechselprodukte. Hinsichtlich der anzuwendenden Menge und der Kosten pro t Bodenmaterial ist dieses Mittel weitaus günstiger als die bisher bekannten Präparate. Die Einsatzmengen richten sich jeweils nach Schadstoffart und -konzentration. Die Ausbringung des Mittels kann z.B. in situ mit den üblichen Beregnungssystemen erfolgen.
Die verwendete entzuckerte Melasselösung fällt als Abfallprodukt bei der Spiritusherstellung aus Zuckerrübenmelasse an. Diese als Vinasse bezeichnete Melasse enthält zahlreiche Spurenelemente (Zn 70 ppm, B 300 ppm, Mn 30 ppm, Mo 1 ppm), die Wachstumsfaktoren für Mikroorganismen sind.
Außerdem enthält sie 2 bis 3 % Saccharose und einen mittleren Gehalt von 3,5 % Gesamtstickstoff. Ein Zusatz an Gesamtstickstoff in Form von 0,28 g Caseintrypton pro g Vinasse verbessert die Biomassebildung wesentlich. Die Biomassebildung sinkt aber bei mehr als 2,5 % organischer Masse schnell auf Grund der fehlenden Energiequelle ab. Die maximale Biomasseproduktion liegt bei 1 % organischer Masse und beträgt 2,1 g BTM/l. Nach Zusatz von 0,28 g Caseintrypton pro g Vinasse beträgt die maximale Biomasseproduktion bei 2,5 % organischer Masse 3,9 g BTM/l.
Als Stickstoffquelle sind erfindungsgemäß pankreatisch abgebaute Proteine geeignet. Diese sind tierischer oder pflanzlicher Herkunft, oder bestehen aus Hefe. Ein hervorragend geeignetes Protein ist das Caseintrypton.
Für den mikrobiellen Abbau von PAK und chlorierten organischen Schadstoffen (Pestizide) ist diese Form der Stickstoffquelle eine wichtige Voraussetzung für einen schnellen Abbau. Um die pH-Schwankungen während der Dekontamination zu kompensieren, wird ein wasserlösliches Phosphat, z.B. Na₂HPO₄ · 12 H₂O, zugesetzt, das gleichzeitig als wichtige Nährstoffquelle dient. Das erfindungsgemäße Mittel stellt eine Nährstoffkombination mit niedrigem Saccharosegehalt als C-Lieferant dar.
Als C-Lieferant fungieren die organischen Schadstoffe, die dann mit der N- und P-Quelle sowie den Vitaminen und Spurenelementen eine hohe Biomasse ergeben.

Dieser Biokomplex enthält ein deutlich vermindertes C-Angebot, so daß die Mikroorganismen als C-Quelle die organischen Schadstoffe nutzen und auf diese Weise deren Abbau bewirkt wird.

Durch ein schnelles Erreichen einer hohen Mikroorganismendichte wird der Schadstoffabbau beschleunigt und für bestimmte Schadstoffe überhaupt erst ermöglicht. Das vorgeschlagene neue Mittel ist ein flüssiges, mit Wasser in jedem Verhältnis mischbares, lagerstabiles Wirkstoffkonzentrat auf der Basis organischer und anorganischer Rohstoffe. Es hat eine Nährstoff-Funktion für Mikroorganismen und ist Stickstoff-, Phosphat-, Vitamin- und Spurenelemente-Quelle und verfügt nur über ein geringes Kohlenstoffangebot.
Durch den Einsatz dieses Mittels werden folgende Bakterienkonzentrationen gebildet. Die BTM/l bezieht sich auf 20 % Bodenfeuchtigkeit und als Teststamm wurde Pseudomonas aeruginosa verwendet.
- 1 · 10¹⁰ KBE/g = 2,6 g: BTM/l mit 120 g
- 1 · 10⁹ KBE/g = 0,26 g: BTM/l mit 12 g
- 1 · 10⁸ KBE/g = 0,026 g: BTM/l mit 1,2 g
- 1 · 10⁷ KBE/g = 0,0026 g: BTM/l mit 0,12 g

Ein weiterer Vorteil besteht in dem sehr günstigen Preis/Leistungsverhältnis. Um eine Bakterienkonzentration von 10⁸ KBE/g Boden (Kompost) zu erzielen, werden 1,2 kg/m³ dieses Biokomplexes benötigt. Die Materialkosten betragen ca. 1 bis 3 DM/m³, so daß auch großflächige Dekontaminationen noch wirtschaftlich entsorgt werden können.
Das erfindungsgemäße Verfahren ist vielseitig anwendbar, z.B. zur Kompostierung und Dekontaminierung von Holzabfällen, Dekontaminierung von Böden, Mauerwerk und Abwässern. Auch zur Entsorgung großflächig ölverschmutzter Wasserflächen ist dieses Verfahren geeignet. Es ermöglicht die Sanierung von mit schwer abbaubaren Schadstoffen, wie z.B. PAK, Chlorphenolen, Nitrophenolen, PCB, kontaminierten Standorten unter Berücksichtigung wirtschaftlicher Aspekte.

Die Erfindung soll nachstehend an mehreren Beispielen erläutert werden.

### Beispiel 1 In situ-Sanierung von verseuchten landwirtschaftlichen Flächen.

Die gezogenen Bodenproben ergaben folgende Schadstoffbelastungen:

| | Rhizosphäre mg/kg TS | Unterboden mg/kg TS |
|---|---|---|
| HCH | 23 | 11 |
| PCB | 10 | 7 |
| Pb | 0,39 | 0,37 |
| Zn | 0,40 | 0,24 |
| Ni | 0,15 | 0,16 |
| Cu | 0,13 | 0,12 |
| Cr | 0,13 | 0,19 |
| (TS bedeutet Trockensubstanz) | | |

Nährstoffe konnten wie folgt nachgewiesen werden:
N: 0,52 % TS; P₂O₅: 1,72 % TS; K: 0 % TS; Mg: 9,33 % TS. Die mikrobiologische Analyse ergab 3,4 · 10⁵ KBE/g Boden.
Die vorhandenen Mikroorganismen wurden mit Azotobacter ergänzt. Diese Bakterien erhöhen die Gesamtmikroorganismen schnell und wirksam. Sie verbessern außerdem den N-Gehalt im Boden und schaffen die Voraussetzung für eine reiche Mikroflora. Danach wird das Erdreich mit einem Biokomplex besprüht, der wie folgt zusammengesetzt ist:

| | |
|---|---|
| entzuckerte Melasselösung vom Typ Vinasse | 65,4 Gew.-% (20g/l) |
| Caseintrypton | 18,3 Gew.-% (5,6 g/l) |
| Na₂HPO₄ · 12 H₂O | 16,3 Gew.-% (5 g/l) |

Pro m³ zu sanierendem Erdboden wurden 1,2 kg dieses Biokomplexes auf die landwirtschaftliche Fläche aufgesprüht, wobei darauf zu achten ist, daß die Spritzbrühe gleichmäßig aufgetragen wird. Die Arbeitstemperatur betrug 20° C und die Bodenfeuchtigkeit 20 %. Durch Kalkzusatz wurde der Erdboden auf einen pH-Wert von 7 eingestellt.
Nach einer Zeitdauer von 2 Monaten wurde der Erdboden erneut mit der gleichen Aufwandsmenge von 1,2 kg/m³ des Biokomplexes besprüht.

Nach 5 Monaten wurden Bodenproben genommen und die Schadstoffbelastung bestimmt, wobei folgende Ergebnisse erzielt wurden:

| | Rhizosphäre (mg/kg TS) | Unterboden (mg/kg TS) |
|---|---|---|
| HCH | 1 | 1 |
| PCB | 0,9 | 0,7 |

Die vorhandenen Mikroorganismen wurden durch den Biokomplex auf 3 · 10⁸ KBE/g TS angehoben.
Der Gehalt an Schwermetallen war nach Anhebung des pH-Wertes durch Kalkung geringfügig niedriger.

### Beispiel 2 Entsorgung von kontaminierten Eisenbahnholzschwellen.

Die Eisenbahnholzschwellen weisen folgende Schadstoffbelastungen auf:

| | | |
|---|---|---|
| AOX | mg Cl/kg TS | 16,2 |
| KW | mg/kg TS | 360 |
| PAK | mg/kg TS | 17,5 |

Die Entsorgung der kontaminierten Eisenbahnholzschwellen erfolgt durch Kompostierung. Der Kompostierungsansatz zur Rotte ist folgender:
350 m³ geschredderte Eisenbahnholzschwellen
50 m³ natürlicher Kompost mit einem hohen Anteil an Zellulosezersetzern
50 m³ Mutterboden
50 m³ strohhaltiger Rinderstalldung.

Die Mikroorganismenkonzentration dieses Ansatzes betrug 6.2 · 10⁷ KBE/g. Nach einer Zeitdauer von 0,5 Monaten wurde der Kompostierungsansatz mit einer Menge von 500 kg eines Biokomplexes folgender Zusammensetzung versetzt:

| | |
|---|---|
| entzuckerte Melasselösung vom Typ Vinasse | 73,9 Gew.-% (22,6 g/l) |
| Caseintrypton | 9,8 Gew.-% (3 g/l) |
| Na₂HPO₄ · 12H₂0 | 16,3 Gew.-% (5,0 g/l) |

Nach einer Zeitdauer von 1,5 Monaten wurde der Kompostierungsansatz erneut mit einer Menge von 2 kg dieses Biokomplexes versetzt. Der erfindungsgemäße Biokomplex wurde jeweils mit Wasser in das Kompostmaterial eingearbeitet. Die Feuchtigkeit im Kompost betrug ca. 50 ± 5 %.
Nach einer Zeitdauer von 5 Monaten hat sich die Anzahl der Mikroorganismen auf 8 · 10¹⁰ KBE/g erhöht.
Die Kompostierung in offener Miete erfolgte über eine Zeitdauer von 7,5 Monaten. Nach 7,5 Monaten wurden folgende Schadstoffkonzentrationen ermittelt:

| | |
|---|---|
| AOX mg Cl/kg TS | < 1 |
| KW mg/kg TS | 238 |
| PAK mg/kg TS | 11 |

Innerhalb dieser Zeitdauer von 7,5 Monaten erfolgte eine erhebliche Schadstoffreduzierung des kontaminierten Holzes und der angefallene Kompost entsprach den vorgeschiebenen Anforderungen als weiterverwertbares Rekultivierungsmaterial.
Während des Kompostierungsverlaufes wurde der Schadstoffabbau überwacht.

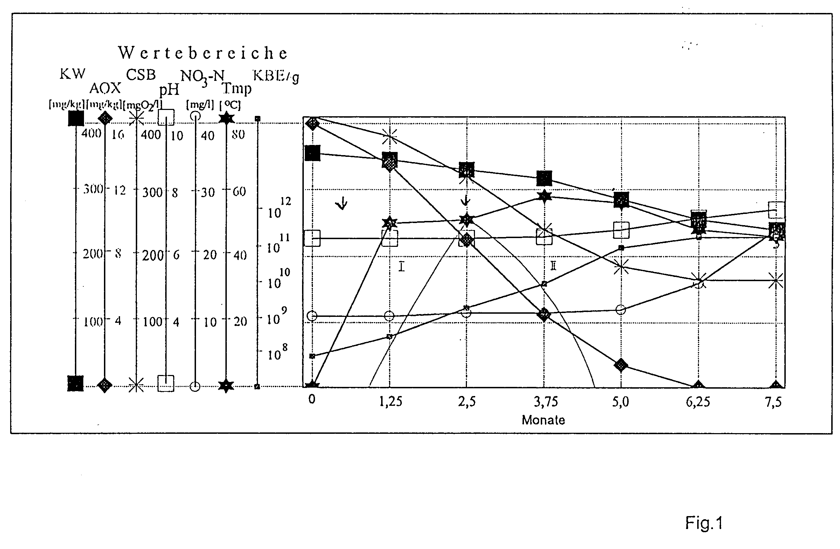

Die Figur 1 zeigt die Stoffumsatzdynamik während der Kompostierung von kontaminierten Eisenbahnholzschwellen. Dargestellt sind der Temperaturverlauf, die bakterielle Mischflora I, die bakteriell-pilzliche Mischflora II sowie der Schadstoffabbau während der Kompostierung. Die einzelnen, gesondert gekennzeichneten Kurven haben dabei folgende Bedeutung:

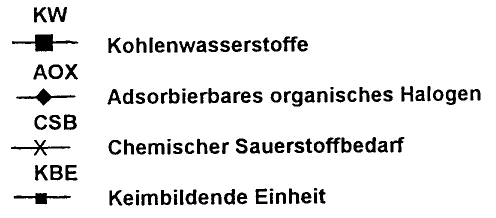

Die mit einem Pfeil ↓ gekennzeichneten Stellen bedeuten, daß zu diesen Zeitpunkten entzuckerte Melasse zugegeben wurde.
Die Diagramme zeigen eine hohe Anfangsschadstoffkonzentration. Die Gesamtschadstoffbelastung, ausgedrückt als CSB, beträgt am Anfang 41 mg O₂/l. Am Ende der Kompostierung, nach 7,5 Monaten, lag sie bei 165 mg O₂/l. Die biologische CSB-Eliminierung betrug daher durch die Kompostierung 60 %. Die Belastung mit Kohlenwasserstoffen betrug zu Beginn der Kompostierung 360 mg/kg TS. Sie wurde zu 33 % abgebaut. Der Kompost entsprach somit den Richtlinien von 1993 für das Land Sachsen-Anhalt. Danach sind für PAK 15 mg/kg TS, AOX 10 mg/kg TS und KW 500 mg/kg TS als zulässig erlaubt.

### Beispiel 3 Entsorgung von kontaminierten Eisenbahnholzschwellen und mineralölhaltigem Schluff durch Kompostierung.

Der Kompostierungsansatz hat folgende Zusammensetzung:
- 350 m³: geschredderte Eisenbahnholzschwellen
- 50 m³: natürlicher Kompost mit einem hohen Anteil an Zellulosezersetzern
- 50 m³: mineralölhaltiger Schluff in pastöser Konsistenz
- 50 m³: strohhaltiger Rinderstalldung

Der Kompostierungsansatz wies folgende Schadstoffbelastung auf:

| | |
|---|---|
| KW mg/kg TS | 978 |
| PAK mg/kg TS | 32 |

Die Anzahl der Mikroorganismen des Kompostierungsansatzes betrug 10⁶ KBE/g.
Zu Beginn der Kompostierung wurden dem Kompostierungsansatz 500 kg des erfindungsgemäßen Biokomplexes folgender Zusammensetzung zugegeben:

| | |
|---|---|
| entzuckerte Melasselösung vom Typ Vinasse | 71,9 Gew.-% (27 g/l) |
| Caseintrypton | 18,3 Gew.-% (5,6 g/l) |
| Na₂HPO₄ · 12H₂0 | 9,8 Gew.-% (3,0 g/l) |

Nach einer Zeitdauer von 2 Monaten wurde erneut die gleiche Menge dieses Biokomplexes zugegeben. Die Einarbeitung erfolgte jeweils mit Wasser und die Feuchtigkeit im Kompost betrug 50 ± 5 %.
Nach einer Zeitdauer von 4 Monaten war der Kompostierungsvorgang abgeschlossen und es wurden folgende Schadstoffe gemessen:

| | |
|---|---|
| KW mg/kg TS | 479 |
| PAK mg/kg TS | 15 |

Die Anzahl der Mikroorganismen im Kompost betrug nach 4 Monaten 2 · 10⁸ KBE/g.

Das Kompostierungsmaterial entsprach den Anforderungen als weiterverwertbares Rekultivierungsmaterial.

### Beispiel 4 In situ-Entsorgung von Erdbodenmaterial, das mit Hydrauliköl verseucht ist.

Die Analyse des Erdbodenmaterials ergab eine Schadstoffbelastung von 6 g KW/kg. Die Mikroorganismendichte betrug 8 · 10³ KBE/g. Die Fläche des zu entsorgenden Erdbodens beträgt 875 m² mit einer Tiefe von 9 m.
Zur in situ-Sanierung wurde ein Biokomplex folgender Zusammensetzung verwendet:

| | |
|---|---|
| entzuckerte Melasselösung vom Typ Vinasse | 65,4 Gew.-% (20 g/l) |
| Caseintrypton | 18,3 Gew.-% (5,6 g/l) |
| Na₂HPO₄ · 12H₂0 | 16,3 Gew.-% (5 g/l) |

Der Biokomplex wurde in einer Menge von 16kg/t Erdboden mit Wasser verdünnt und über eine Berieselungsanlage gleichmäßig auf die zu sanierende Fläche aufgebracht.
Durch den Zusatz dieses Biokomplexes erhöhte sich die Mikroorganismendichte auf 10⁸ KBE/g. Die Bodenfeuchtigkeit wurde auf einen Wert von 40 ± 5 % eingestellt.
Während der Sanierung betrug die durchschnittliche Bodentemperatur 18° C.
Nach einer Zeitdauer von 44 Tagen konnte im sanierten Boden eine erhebliche Schadstoffreduzierung festgestellt werden. Die Analyse ergab eine Belastung von 455 mg KW/kg Boden. Diese sehr geringe Schadstoffbelastung, die bereits nach 44 Tagen Behandlungszeit erreicht wurde, stellt keine Gefährdung mehr für das Grundwasser dar.

### Beispiel 5 Dechlorierung von chlorphenolhaltigem Wasser aus der Bodenwäsche im Fermenter.

Chlorphenolhaltiges Bauschuttgranulat wurde mit 1 %iger Sodalösung gewaschen. Das angefallene Waschwasser hat einen AOX-Wert von 323 mg Cl/l.
Die Dechlorierung des mit Chlorphenol kontaminierten Wassers im Fermenter erfolgte unter folgenden Bedingungen:
- - Betriebstemperatur: 30° C
- - Drehzahl: 300 U/min
- - Volumen: 300 l
- - Fermentationszeit: 5 Tage

Im Fermenter sind bodenständige Bakterien Pseudomonas aeruginosa in einer Menge von 5 l mit 3 · 10⁹ KBE/g eingebracht und pro Liter chlorphenolhaltigem Wasser werden 30,6 g eines Biokomplexes folgender Zusammensetzung und 2g KNO₃ als Akzeptor in den Fermenter eingetragen. Zusammensetzung des Biokomplexes:

| | |
|---|---|
| entzuckerte Melasselösung vom Typ Vinasse | 65,4 Gew.-% (20 g/l) |
| Caseintrypton | 18,3 Gew.-% (5,6 g/l) |
| Na₂HPO₄ · 12H₂0 | 16,3 Gew.-% (5 g/l) |

Es wurde eine Mikroorganismendichte von 10⁹ KBE/g erreicht (2,17 BTM/l). Nach einer Fermentationszeit von 5 Tagen wurde das behandelte Wasser analysiert und der gemessene AOX-Wert betrug nur noch 0,8 mg/l.
Infolge dieser geringen Schadstoffkonzentration des Wassers konnte dieses in das örtliche Abwassernetz eingeleitet werden.

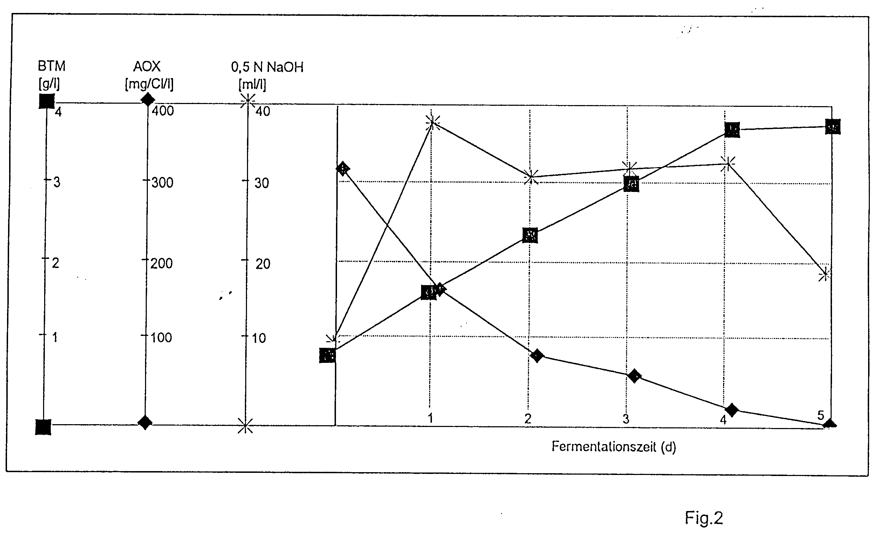

In der Figur 2 ist die Stoffumsatzdynamik während der mikrobiellen Dechlorierung von Chlorphenol durch Pseudomonas aerugionsa im Fermenter dargestellt. Dabei bedeuten BTM die Bakterientrockenmasse und AOX Adsorbierbares organisches Halogen.

## Patentansprüche

1. Verfahren zur Dekontaminierung von in Feststoffen oder Flüssigkeiten enthaltenen organischen Schadstoffen mittels Mikroorganismen, dadurch gekennzeichnet, daß dem kontaminerten Stoff ein Mittel bestehend aus
a) 3 bis 26 Gew.-% pankreatisch abgebauter Proteine,
b) 3 bis 20 Gew.-% P₂O₅ enthaltende wasserlösliche Phosphate und
c) als Rest entzuckerte Melasselösung mit weniger als 50 g/l Zucker (Invertzucker) und mehr als 30 g/l Stickstoff sowie Vitaminen und Spurenelementen,
ein- oder mehrmals zugesetzt wird.

2. Verfahren nach Anspruch 1, dadurch gekennzeichnet, daß die Anzahl der Mikroorganismen mindestens 10³ KBE/g beträgt.

3. Verfahren nach Anspruch 1 oder 2, dadurch gekennzeichnet, daß durch Zugabe weiterer Mikroorganismen und/oder speziell angezüchteter Spezies als Animpfmaterial die Anzahl der Mikroorganismen auf mindestens 10⁷ KBE/g erhöht wird.

4. Verfahren nach Anspruch 3, dadurch gekennzeichnet, daß die Spezies Pseudomonas, Azotobacter, Flavobakterien, Penicillium, Schleimpilze, Alcaligenes, Weißfäulepilze oder Chlostridium sind.

5. Verfahren nach einem der Ansprüche 1 bis 4, dadurch gekennzeichnet, daß die Einsatzmenge des Mittels in Abhängigkeit von der Anzahl der Mikroorganismen variiert wird, wobei sich mit zunehmender Anzahl an Mikroorganismen die Einsatzmenge verringert, und die Einsatzmenge in einem Bereich von 1 bis 10 kg/m³ kontaminiertem Stoff liegt.

6. Verfahren nach einem der Ansprüche 1 bis 5, dadurch gekennzeichnet, daß die pankreatisch abgebauten Proteine tierischer oder pflanzlicher Herkunft sind, oder aus Hefe bestehen.

7. Verfahren nach einem der Ansprüche 1 bis 6, dadurch gekennzeichnet, daß die entzuckerte Melasselösung aus 48 Gew.-% organischer Masse, 2 bis 3 Gew.-% Zucker, 3,5 Gew.-% Stickstoff und als Rest Wasser besteht und als Spurenelemente Bor, Kupfer, Mangan, Molybdän und Zink enthält.

8. Verfahren nach einem der Ansprüche 1 bis 7, dadurch gekennzeichnet, daß die Melasselösung mit Saccharomyces entzuckert ist.

9. Verfahren nach einem der Ansprüche 1 bis 8, dadurch gekennzeichnet, daß als Animpfmaterial natürlicher Kompost mit einem hohen Anteil an Zellulosezersetzern und/oder strohhaltiger Stalldung und/oder entwässerter Klärschlamm zugesetzt werden.

10. Verfahren nach Anspruch 9, dadurch gekennzeichnet, daß das Animpfmaterial in einer Menge von 30 bis 50 Gew.-%, bezogen auf die Einsatzmenge an kontaminiertem Stoff, zugegeben wird.

11. Verfahren nach einem der Ansprüche 1 bis 10, dadurch gekennzeichnet, daß dieses bei Arbeitstemperaturen von 10 bis 40° C, einem Feuchtigkeitsgehalt von 20 bis 60 % und einem pH-Wert von 6 bis 8 des kontaminierten Stoffes unter aeroben oder anaeroben Bedingungen durchgeführt wird.

12. Verfahren nach einem der Ansprüche 1 bis 11, dadurch gekennzeichnet, daß die Dekontaminierung als Kompostierung in Verbindung mit organischem Material oder in erdigen Substanzen in situ oder in Biobeeten oder in wäßriger Lösung im Fermenter oder in flüssiger Phase durchgeführt wird.

13. Mittel zur Durchführung des Verfahrens nach Anspruch 1, dadurch gekennzeichnet, daß dieses aus
a) 3 bis 26 Gew.-% pankreatisch abgebauter Proteine,
b) 3 bis 20 Gew.-% P₂O₅ enthaltende wasserlösliche Phosphate und
c) als Rest entzuckerte Melasselösung mit weniger als 50 g/l Zucker (Invertzucker) und mehr als 30 g/l Stickstoff, sowie Vitaminen und Spurenelementen,
als Biokomplex besteht.

14. Mittel nach Anspruch 13, dadurch gekennzeichnet, daß die pankreatisch abgebauten Proteine tierischer oder pflanzlicher Herkunft sind, oder aus Hefe bestehen.

15. Mittel nach einem der Ansprüche 13 oder 14, dadurch gekennzeichnet, daß die entzuckerte Melasselösung aus 48 Gew.-% organischer Masse, 2 bis 3 Gew.-% Zucker, 3,5 Gew.-% Stickstoff und als Rest Wasser besteht und als Spurenelemente Bor, Kupfer, Mangan, Molybdän und Zink enthält.

16. Mittel nach einem der Ansprüche 13 bis 15, dadurch gekennzeichnet, daß die Melasselösung mit Saccharomyces entzuckert ist.

17. Verwendung eines Mittels nach Anspruch 13 zur Entsorgung von ölverschmutzten Wasserflächen.

## Claims

1. Process for the decontamination or organic noxious substances contained in solids or liquids by means of micro-organisms, characterised by the fact that to the contaminated substance is added, once or a number of times, an agent consisting or:
(a) 3 to 26% by weight of pancreatically decomposed proteins,
(b) 3 to 20% by weight of water-soluble phosphates containing P₂0₅ and,
(c) molasses solution deprived of sugar, as the remainder, with less than 50 g/l of sugar (invert sugar) and over 30 g/l of nitrogen, together with vitamins and trace elements.

2. Process according to Claim 1, characterised by the fact that the number of micro-organisms amounts to at least 10³ KBE/g.

3. Process according to Claim 1 or 2, characterised by the fact that the number of micro-organisms and/or specially cultivated species as graffing media, the number of micro-organisms is increased to at least 10⁷ KBE/g.

4. Process according to Claim 3, characterised by the fact that the species are pseudomonas, azotobacteria, flavobacteria, penicillium, slime fungi, alkaligenes, white rot fungi or chlostridium.

5. Process according to any one of Claims 1 to 4, characterised by the fact that the quantity of the medium used varies according to the number of micro-organisms, being reduced as the number of microorganisms increases, and the quantity used ranges from 1 to 10 kg/m³ of contaminated substance.

6. Process according to any one of Claims 1 to 5, characterised by the fact that the pancreatically decomposed proteins are of animal or vegetable origin or consist of yeast.

7. Process according to any one of Claims 1 to 6, characterised by the fact that the molasses solution deprived of sugar consists of 48% by weight of organic substance, 2 to 3% by weight of sugar and 3.5% by weight of nitrogen, the remainder being water, and contains boron, copper, manganese, molybdenum and zinc as trace elements.

8. Process according to any one of Claims 1 to 7, characterised by the fact that the molasses solution is deprived of sugar by means of saccharomyces.

9. Process according to any one of Claims 1 to 8, characterised by the fact that the grafting medium added consists of natural compost with a high proportion of cellulose decomposing agents and/or stable manure containing straw and/or dehydrated clarifying sludge.

10. Process according to Claim 9, characterised by the fact that the grafting material is added in a quantity of 30 to 50% by weight based on the quantity of contaminated substance involved.

11. Process according to any one of Claims 1 to 10, characterised by the fact that it is carried out at operating temperatures of 10 to 40° C and with a moisture content of 20 to 60%, the pH value of the contaminated substance being from 6 to 8, under aerobic or anaerobic conditions.

12. Process according to any one of Claims 1 to 11, characterised by the fact that the decontamination is carried out in conjunction with organic material or in earthy substances in situ or in biological beds or in aqueous solution in the fermenter or in the liquid phase.

13. Agent for the performance of the process according to Claim 1, characterised by the fact that the agent consists of:
(a) 3 to 26% by weight of pancreatically decomposed proteins,
(b) 3 to 20% by weight of water-soluble phosphates containing P₂O₅ and
(c) molasses solution deprived of sugar, as the remainder, with less than 50 g/l of sugar (invert sugar) and over 30 g/l of nitrogen, as well as vitamins and trace elements,
as a biological complex.

14. Agent according to Claim 13, characterised by the fact that the pancreatically decomposed proteins are of animal or vegetable origin or consist of yeast.

15. Agent according to either one of Claims 13 and 14, characterised by the fact that the molasses solution deprived of sugar consists of 48% by weight of organic substances, 2-3% by weight of sugar, 3.5% by weight of nitrogen, the remainder consisting of water, with boron, copper, manganese, molybdenum and zinc as trace elements.

16. Agent according to any one of Claims 13 to 15, characterised by the fact that the molasses solution is deprived of sugar by means of saccharomyces.

17. Use of an agent according to Claim 13 for the disposal of oil-contaminated water surfaces.

## Revendications

1. Procédé de décontamination de substances organiques nuisibles contenues dans des solides ou des liquides au moyen de micro-organismes, caractérisé en ce que l'on ajoute une fois ou plusieurs fois au produit contaminé un agent composé de:
a) 3 à 26% en poids de protéines dégradées par voie pancréatique,
b) 3 à 20% en poids de phosphates hydrosolubles contenant du P₂O₅,
c) le reste étant une solution de mélasse désucrée contenant moins de 50 g/l de sucre (sucre inverti) et plus de 30 g/l d'azote, ainsi que des vitamines et des oligo-éléments.

2. Procédé selon la revendication 1, caractérisé en ce que le nombre de micro-organismes est égal à au moins 10³ UFC/g.

3. Procédé selon la revendication 1 ou la revendication 2, caractérisé en ce que le nombre de micro-organismes est augmenté à au moins 10⁷ UFC/g par addition d'autres micro-organismes et/ou d'espèces spécialement cultivées comme matériau d'ensemencement.

4. Procédé selon la revendication 3, caractérisé en ce que les espèces sont Pseudomonas, Azotobacter, Flavobacterium, Penicillium, Myxomycètes, Alcaligenes, Fusarium ou Clostridium.

5. Procédé selon l'une quelconque des revendications 1 à 4, caractérisé en ce que la quantité d'agent utilisée varie en fonction du nombre de micro-organismes, la quantité utilisée diminuant à mesure que le nombre des micro-organismes augmente, et en ce que la quantité utilisée se situe dans la plage de 1 à 10 kg par m³ de produit contamine.

6. Procédé selon l'une quelconque des revendications 1 à 5, caractérisé en ce que les protéines dégradées par voie pancréatique sont d'origine animale ou végétale ou bien se composent de levure.

7. Procédé selon l'une quelconque des revendications 1 à 6, caractérisé en ce que la solution de mélasse désucrée se compose de 48% en poids de masse organique, de 2 à 3% en poids de sucre, de 3,5% en poids d'azote, le reste étant de l'eau, et contient comme oligo-éléments du bore, du cuivre, du manganèse, du molybdène et du zinc.

8. Procédé selon l'une quelconque des revendications 1 à 7, caractérisé en ce que la solution de mélasse est désucrée avec Saccharomyces.

9. Procédé selon l'une quelconque des revendications 1 à 8, caractérisé en ce que le matériau d'ensemencement ajouté est un compost naturel ayant une teneur élevée en agents de décomposition de la cellulose et/ou du fumier d'étable contenant de la paille et/ou une boue d'épuration déshydratée.

10. Procédé selon la revendication 9, caractérisé en ce que le matériau d'ensemencement est ajouté à raison de 30 à 50% en poids ramené à la quantité de produit contaminé utilisée.

11. Procédé selon l'une quelconque des revendications 1 à 10, caractérisé en ce que ledit procédé est mis en oeuvre à une température de travail de 10 à 40°C, une teneur en humidité de 20 à 60% et une valeur de pH de 6 à 8 pour le produit contaminé en conditions aérobies ou anaérobies.

12. Procédé selon l'une quelconque des revendications 1 à 11, caractérisé en ce que la décomposition se fait sous la forme d'un compostage en liaison avec un matériau organique ou dans des substances du sol in situ ou dans des planches biologiques ou dans une solution aqueuse dans un fermenteur ou dans une phase liquide.

13. Agent permettant de mettre en oeuvre le procédé selon la revendication 1, caractérisé en ce que ledit agent se compose de:
a) 3 à 26% en poids de protéines dégradées par voie pancréatique,
b) 3 à 20% en poids de phosphates hydrosolubles contenant du P₂O₅,
c) le reste étant une solution de mélasse désucrée contenant moins de 50 g/l de sucre (sucre inverti) et plus de 30 g/l d'azote, ainsi que des vitamines et des oligo-éléments,
sous la forme d'un complexe biologique.

14. Agent selon la revendication 13, caractérisé en ce que les protéines dégradées par voie pancréatique sont d'origine animale ou végétale, ou bien se composent de levure.

15. Agent selon la revendication 13 ou la revendication 14, caractérisé en ce que la solution de mélasse désucrée se compose de 48% en poids de masse organique, de 2 à 3% en poids de sucre, de 3,5% en poids d'azote, le reste étant de l'eau, et contient comme oligo-éléments du bore, du cuivre, du manganèse, du molybdène et du zinc.

16. Agent selon l'une quelconque des revendications 13 à 15, caractérisé en ce que la solution de mélasse est désucrée avec Saccharomyces.

17. Utilisation d'un agent selon la revendication 13 pour traiter des surfaces d'eau contaminées par des huiles.
